(19) Europäisches Patentamt
European Patent Office
Office européen des brevets

(11) Publication number: **0 150 974 A2**

# (12) EUROPEAN PATENT APPLICATION

(21) Application number: **85300413.3**

(22) Date of filing: **22.01.85**

(51) Int. Cl.⁴: **C 07 D 487/04**, A 01 N 43/90
// (C07D487/04, 249:00, 239:00)

(30) Priority: **26.01.84 US 574232**

(43) Date of publication of application: **07.08.85 Bulletin 85/32**

(84) Designated Contracting States: **AT BE CH DE FR GB IT LI NL SE**

(71) Applicant: **THE DOW CHEMICAL COMPANY, Dow Center 2030 Abbott Road Post Office Box 1967, Midland Michigan 48640 (US)**

(72) Inventor: **Kleschick, William A., 640 Fig Tree Lane Martinez, Contra Costa California 94553 (US)**

(74) Representative: **Allard, Susan Joyce et al, BOULT, WADE & TENNANT 27 Furnival street, London EC4A 1PQ (GB)**

(54) **Novel sulfonamides derived from substituted 2-amino-1,2,4-triazolo(1,5-a)pyrimidines and compositions and methods of controlling undesired vegetation.**

(57) Novel compounds, e.g., N-(5,7-dimethyl-1,2,4-triazolo-[1,5-a]pyrimidin-2-yl)-2-thiophene sulfonamide and their compositions and use in the control of weeds.

EP 0 150 974 A2

# NOVEL SULFONAMIDES DERIVED FROM SUBSTITUTED 2-AMINO-1,2,4-TRIAZOLO[1,5-a]PYRIMIDINES AND COMPOSITIONS AND METHODS OF CONTROLLING UNDESIRED VEGETATION

In recent years there has been a great deal of effort directed to the development of sulfonamides having herbicidal activity and several of these compounds have reached the stage of commercialization, i.e., chlorosulfuron and sulfometuron methyl. These compounds exhibit both preemergence and postemergence activity against undesirable vegetation and, in addition, have a low toxicity to mammals. The compounds of the prior art may be depicted as follows:

$$Ar\text{-}SO_2NH\overset{O}{\overset{\|}{C}}NH\text{-}Ar'$$

wherein Ar is usually a benzene derivative and Ar' is usually a pyrimidine or symmetrical triazine derivative.

In addition, there are a number of other sulfonamide herbicides that have been commercialized, for example, methyl sulfanilylcarbamate; O,O-diisopropyl

phosphorodithioate-S-ester with N-(2-mercaptoethyl)-benzenesulfonamide; 3-isopropyl-1H-2,1,3-benzothiadiazin-4(3H)-one 2,2-dioxide; N-[2,4-dimethyl-5-[[(trifluoromethyl)sulfonyl]amino]phenyl]acetamide; 3,5-dinitro-$N^4$,$N^4$-dipropylsulfanilamide and 2-t-butyl-4-(2,4-dichloro-5-isopropoxyphenyl)-$\Delta^2$-1,3,4-oxadiazolin-5-one.

We have now found that compounds having the formula:

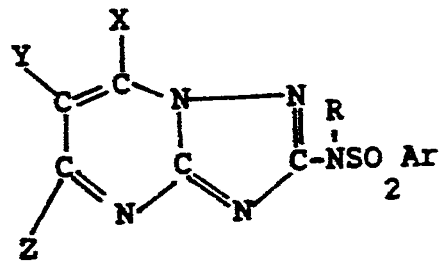

wherein Ar represents a substituted or unsubstituted aromatic or heteroaromatic ring system, R represents hydrogen, alkyl, alkenyl, alkynyl, arylalkyl, substituted aralkyl, acyl, alkoxycarbonyl, aryloxycarbonyl, dialkylaminocarbonyl, alkylsulfonyl, arylsulfonyl, alkylthiocarbonyl or arylthiocarbonyl and X, Y and Z independently represent hydrogen, alkyl, haloalkyl, hydroxy, alkoxy, haloalkoxy, aryl, substituted aryl, halogen, alkylthio, amino, carboxylic acids and esters or two adjacent substituents are joined together in a cyclic structure, or the pyrimidine ring may be reduced to form a 4,5,6,7-tetrahydro-1,2,4-triazolo[1,5-a]pyrimidine ring; are exceptionally active herbicides.

The aromatic or heteroaromatic ring systems include, for example, phenyl; 1- or 2-napthyl; 2-, 3- or 4-pyridyl; 2- or 3-thienyl; 2- or 3-furyl; 2-, 4- or 5-thiazolyl; 2-, 4- or 5-imidazolyl; 2-, 4- or 5-oxazolyl;

3-, 4- or 5-isothiazolyl; 3-, 4- or 5-isoxazolyl; 3-, 4- or 5-pyrazolyl; 2-benzthiazolyl; 2-benzoxazolyl; 2-benzimidiazolyl and 1-benztriazolyl. Typical examples of substituents found on the aromatic or heteroaromatic ring systems may be one, more than one or a combination of the following: halo (F, Cl, Br, I), alkyl, haloalkyl, aryl, hydroxy, alkoxy, haloalkoxy, aryloxy, substituted aryloxy, heteroaryloxy, substituted heteroaryloxy, amino, alkylamino, dialkylamino, nitro, alkylthio, haloalkylthio, alkylsulfinyl, haloalkylsulfinyl, alkylsulfonyl, haloalkylsulfonyl, substituted or unsubstituted arylthio, substituted or unsubstituted arylsulfinyl, substituted or unsubstituted arylsulfonyl, cyano, carboxylic acids (and derivatives of carboxylic acids such as esters derived from readily available alcohols and amides derived from ammonia or readily available primary and secondary amines), sulfonic acids (and derivatives of sulfonic acids such as sulfonates derived from readily available alcohols and sulfonamides derived from ammonia or readily available primary or secondary amines), formyl, alkylcarbonyl, haloalkylcarbonyl, arylcarbonyl, substituted arylcarbonyl, oximino, oxime ethers, carbinols (and carbinol derivatives such as ethers and esters derived from readily available alkylating agents and carboxylic acids respectively) and mercaptoalkyl (and derivatives of mercaptoalkyl groups such as thioethers and thioesters derived from readily available alkylating agents and carboxylic acids respectively).

The substituents on the triazolopyrimidine fragment of structure I are represented by X, Y and Z. Substituents X, Y and Z may be H, alkyl, haloalkyl, hydroxy, alkoxy, haloalkoxy, aryl, substituted aryl, halo (F, Cl, Br, I), alkylthio, arylthio, amino (including alkyl or aryl substituted amino), carboxylic acids and

esters. In addition, two adjacent substituents (i.e., X and Y or Y and Z) may be joined together in a cyclic structure. Examples of such cyclic structures could be represented by X, Y or Y, Z equal to $-(CH_2)_n-$ where n = 3, 4 or 5. These cyclic structures may also contain heteroatoms as in the case where X, Y or Y, Z is equal to $-(CH_2)_nO-$ where n = 2 or 3.

Preferred compounds of the invention have the general formula:

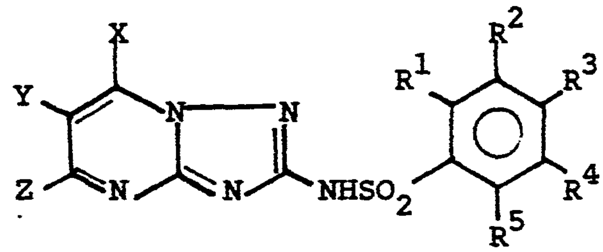

wherein $R^1$ represents halo (F, Cl, Br, I), $-NO_2$, phenyl, OAr, $-CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, -CN, $-COOR^7$, $-CONH_2$, $-CONHR^8$, $-CONR^8(R^9)$, $-SO_3R^8$ and $-SO_3CH_2CF_3$; $R^2$ and $R^4$ represent H, halo (F, Cl, Br, I), $C_1$-$C_4$ alkyl, $COOR^7$ and $-OR^8$; $R^3$ is H; and $R^5$ represents H, $C_1$ to $C_4$ alkyl, halo (F, Cl, Br, I), $NO_2$, $CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, -CN, $-COOR^7$, $-CONH_2$, $-CONHR^8$, $-CONR^8(R^9)$, $-SO_3R^8$, $-SO_3CH_2CF_3$, $-CR^6R^6OR^6$ and $-CR^6R^6SR^6$ wherein Ar represents substituted or unsubstituted aryl or substituted or unsubstituted heteroaryl, $R^6$ represents H, aryl or $C_1$-$C_4$ alkyl, $R^7$

represents $C_1$-$C_6$ alkyl, alkenyl,alkynyl, aryl, substituted alkyl or substituted aryl and $R^8$ and $R^9$ individually represent $C_1$-$C_4$ alkyl; and X, Y and Z represent H, $C_1$-$C_4$ alkyl, $C_1$-$C_4$ alkoxy halo (F, Cl, Br, I), or X and Y or Y and Z can be joined to form a cycloalkyl ring (i.e., $-(CH_2)_n-$ wherein n is 3 or 4) or X and Y or Y and Z can be joined to form a ring containing a heteroatom (i.e., $-O(CH_2)_n$ wherein n is 2 or 3).

Preferred compounds of the invention also have the general formula:

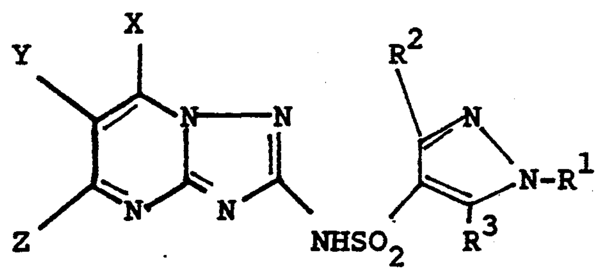

wherein $R^1$ represents H, alkyl or aryl, $R^2$ and $R^3$ represent independently H, $C_1$-$C_4$ alkyl, halo (F, Cl, Br, I), $-NO_2$, phenyl, $-CF_3$, benzyl, $-COOR^4$, $-CONH_2$, $-CONHR^5$, $-CONR^5R^6$, and CN wherein $R^4$ represents $C_1$-$C_6$ alkyl, alkenyl, alkynyl, arylalkyl, substituted alkyl or substituted aryl, $R^5$ and $R^6$ individually represent $C_1$-$C_4$ alkyl; and X, Y and Z represent H, $C_1$-$C_4$ alkyl, $C_1$-$C_4$ alkoxy, halo (F, Cl, Br, I), or X and Y or Y and Z can be joined to form a cycloalkyl ring (i.e., $-(CH_2)_n-$ wherein n is 3 or 4) or X and Y or Y and Z can be joined to form a ring containing a heteroatom (i.e., $-O(CH_2)_n-$ wherein n is 2 or 3.

Most preferred compounds of the invention have the general formula:

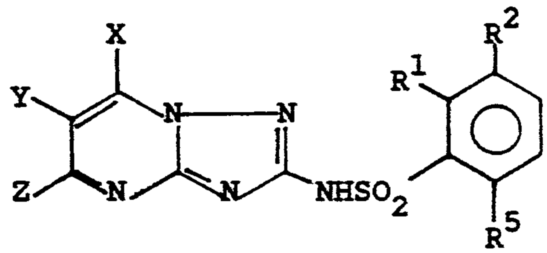

wherein $R^1$ represents $C_1$-$C_4$ alkyl, halo (F, Cl, Br, I), $-NO_2$, $-SR^6$, $-SOR^6$, $SO_2R^6$, $-COOR^7$ or $CF_3$; $R^2$ represents H, halo (F, Cl, Br, I), $C_1$-$C_4$ alkyl, and $COOR^7$; and $R^5$ represents H, $C_1$-$C_4$ alkyl, halo (F, Cl, Br, I), $CH_2OR^6$, phenyl, $NO_2$ and $COOR^7$ wherein $R^6$ represents $C_1$-$C_4$ alkyl and $R^7$ represents $C_1$-$C_4$ alkyl, $C_1$-$C_4$ alkenyl, $C_1$-$C_4$ alkynyl, 2-ethoxyethyl and 2-pyridylmethyl and X, Y and Z independently represent H, halo (F, Cl, Br, I), $C_1$-$C_4$ alkyl or $C_1$-$C_4$ alkoxy.

In addition certain derivatives of compounds corresponding to I also exhibit herbicidal activity. For example, compounds having the formula:

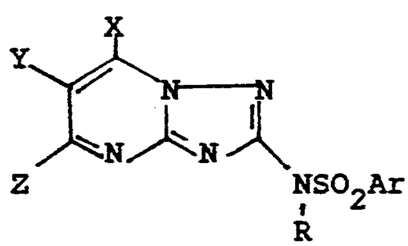

(II)

wherein Ar and X, Y and Z are as described above for compound I and R represents alkyl, alkenyl, alkynyl,

arylalkyl, substituted arylalkyl, acyl, alkoxycarbonyl, aryloxycarbonyl, dialkylaminocarbonyl, alkylsulfonyl, arylsulfonyl, alkylthiocarbonyl or arylthiocarbonyl.

Preferred derivatives of the invention have the general formula:

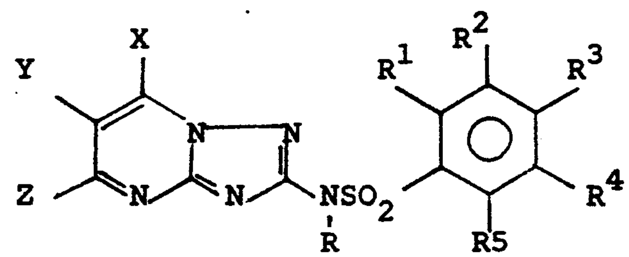

wherein X, Y, Z, $R^1$, $R^2$, $R^3$, $R^4$ and $R^5$ are as described above for I and R represents $C_1$-$C_4$ alkyl, allyl, benzyl, $-COR^{10}$, $-CO_2R^{10}$, $-CONR_2{}^{10}$, $-COSR^{10}$, and $-SO_2R^{10}$ wherein $R^{10}$ is $C_1$-$C_6$ alkyl, aryl, substituted aryl or haloalkyl.

Most preferred derivatives of the invention have the general formula:

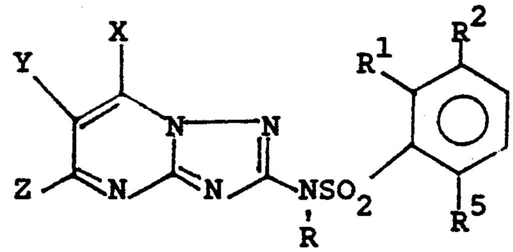

wherein X, Y, Z, $R^1$, $R^2$ and $R^5$ are as described above for I and R is $COR^{10}$ wherein $R^{10}$ is $C_1$-$C_4$ alkyl.

Another series of derivatives of compounds of type I also possess herbicidal activity. These compounds are represented by the general formula:

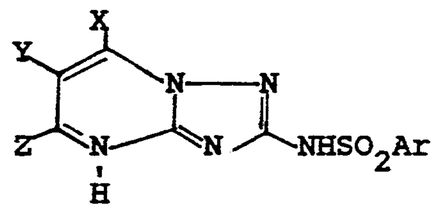

(III)

wherein X, Y, Z and Ar are as described above for compounds of type I.

Preferred derivatives of this invention have the general formula:

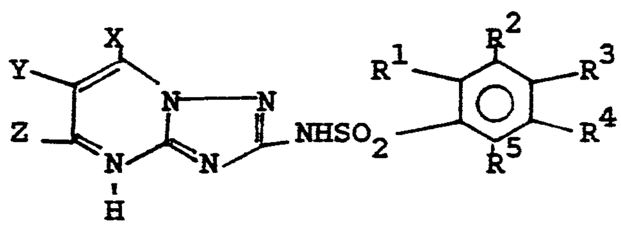

wherein X, Y, Z, $R^1$, $R^2$, $R^3$, $R^4$, and $R^5$ are as described above for I.

Most preferred derivatives of this invention have the general formula:

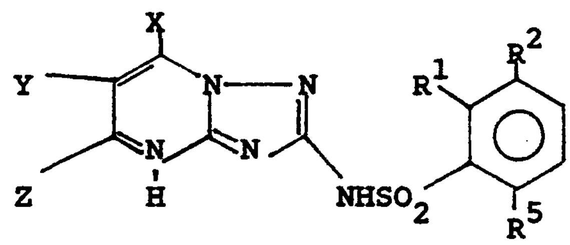

wherein X, Y, Z, $R^1$, $R^2$ and $R^5$ are as described above for I.

Furthermore, in the above invention corresponding to general formula III the existence of stereoisomerism is possible. For example stereoiosmeric relationships exist when at least one of substituents X, Y and Z does not equal hydrogen. When only one of substituents X, Y and Z does not equal hydrogen the compound of type III may exist as a mixture of enantiomers. One enantiomer will be designated as having the R-configuration and the other will be designated as having the S-configuration. Each enantiomer may exhibit different levels of herbicidal activity. When two or more of substituents X, Y or Z in structure III do not equal hydrogen, the material may exist as a mixture of diastereomers. For example when two substituents among X, Y and Z do not equal hydrogen, the compound may exist as two diastereomers. When all three of substituents X, Y and Z do not equal hydrogen the compound may exist as four diastereomers. In addition all of the diastereomers described above exist as a mixture of two enantiomers. All of the stereoisomers described above, diastereomers and their enantiomeric pairs, may exhibit different levels of herbicidal activity.

The synthesis of compounds of general structure I can be carried out in a straightforward manner as illustrated in SchemeI. Reaction of the appropriate aromatic sulfonyl chloride IV with the required 2-amino-1,2,4-triazolo[1,5-a] pyrimidine V under basic conditions yields the desired product I. A wide range of solvents may be employed (i.e., $CH_2Cl_2$, $CH_3CN$ or pyridine) at temperatures ranging from 0°C to reflux. Bases which serve as catalysts include pyridine, 4-dimethylaminopyridine and teritary alkylamines such as triethylamine or N-methylmorpholine. Genreally the amino compound serves as the limiting reagent. Molar ratios of between 1.1 and 1.0 for the sulfonylchloride to amino compound and molar ratios of between 5.0 and 1.1 for the base to amino compound are used most often. A wide range of concentrations may be employed (i.e., 0.1-5M). Generally concentrations in the range of 0.5-2M are used to give a homogeneous reaction which proceeds at a convenient rate. In addition it is sometimes advantageous to use a combination of pyridine derived base catalysts and tertiary amine bases. The use of pyridine as a solvent is convenient as the pyridine can serve both as a solvent and catalyst in the transformation.

<u>SCHEME I</u>

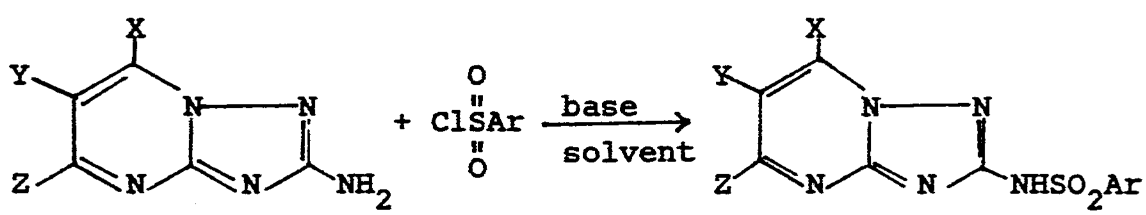


V IV I

The required sulfonyl chlorides II are often commercially available. In some cases sulfonyl chlorides are prepared by the methodology outlined by H. T. Clarke et al., Org. Synth. Coll., Vol. 1, 2nd Ed., 1941, p. 85. This involves chlorosulfonation of the appropriate substituted benzene. Other sulfonyl chlorides can be prepared by methods described by R. V. Hoffman, Org. Synth., Vol. 60, p. 121. This involves diazotization of the appropriate substituted aniline or amino substituted heterocycle with sodium nitrite in acidic media followed by reaction of the diazonium salt with sulfur dioxide in the presence of cuprous chloride. In addition certain sulfonyl chlorides can be prepared from aromatic compounds containing benzylthio groups. The benzylthio functional group is converted to a sulfonyl chloride by treatment with chlorine in aqueous acidic media.

The required substituted 2-amino-1,2,4--triazolo[1,5-a]pyrimidine V can be prepared by methods outlined in "Heterocyclic Systems with Bridgehead Nitrogen Atoms", Part Two, W. L. Mosby, Interscience Publishers, 1961, p. 878. This involves the reaction of the appropriate 1,3-dicarbonyl compound with 3,5-diamino-1,2,4-triazole VI under acidic or basic conditions (Scheme II). The appropriate 1,3-dicarbonyl compounds include substituted 1,3-diketones, malonic esters, malonaldehyde, β-ketoesters, β-ketoaldehydes and α-formyl esters and derivatives thereof (i.e., acetals or enol ethers).

SCHEME II

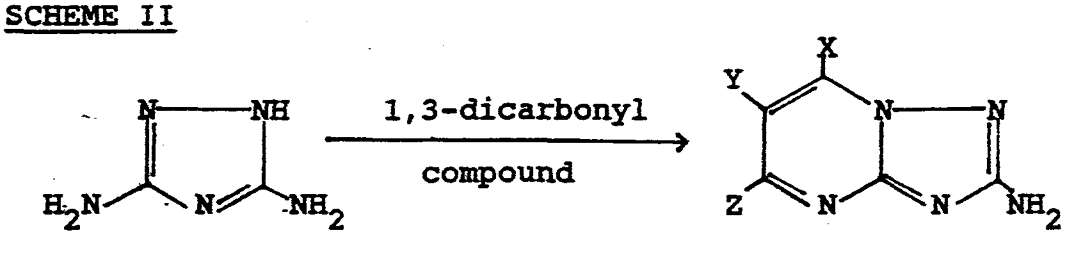


VI V

In instances where the 1,3-dicarbonyl compound is unsymmetrical, the possibility of obtaining two different isomers from condensation with VI exists. In general, under acidic conditions the exocyclic nitrogen in VI is the first to condense with the 1,3-dicarbonyl compound. Under basic conditions the endocyclic nitrogen in VI is more reactive. Consequently, in situations where a clear difference in reactivity of the two carbonyl functionalities in the 1,3-dicarbonyl compound exists, some measures of regiochemical control may be achieved by choice of reaction conditions.

In the synthetic routes listed above, compounds of type V where X and/or Z is OH are capable of undergoing further transformation (Scheme III). For example, treatment of compound V (X and/or Z = OH) with phosphorus oxychloride yields V (X and/or Z = Cl). The reaction is generally carried out at reflux in neat phosphorus oxychloride. Compound V (X and/or Z = Cl) can be further reacted with nucleophiles (i.e., $NaOCH_3$, MeMgBr) to yield V (X and/or Z $OCH_3$ or $CH_3$, respectively).

SCHEME III

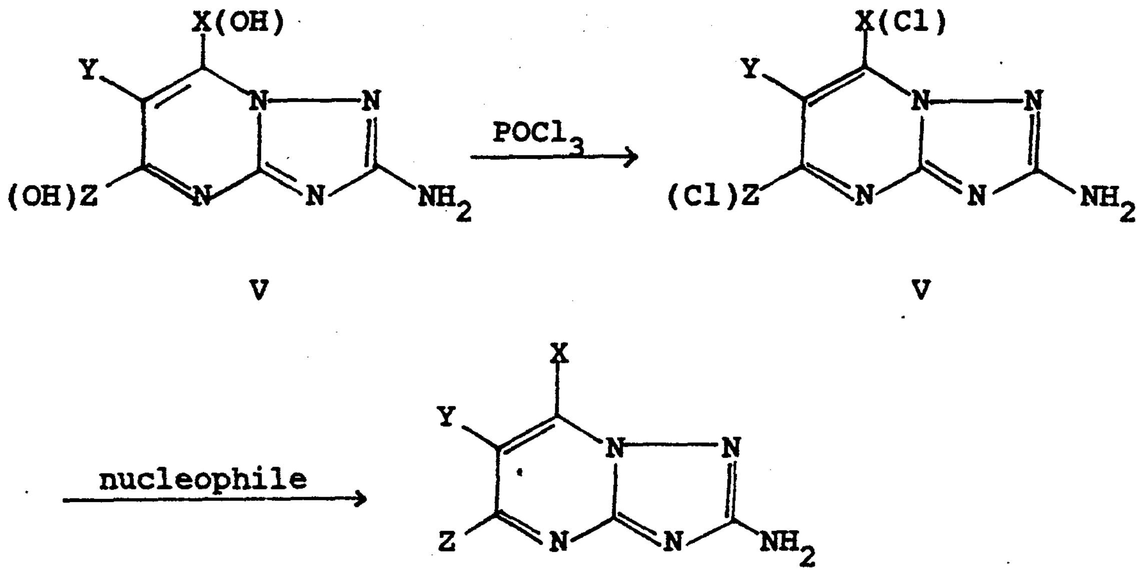


V V

V

Compounds of the present invention represented by structure II are derived from compounds represented by structure I as illustrated in Scheme IV. The derivatization procedure involves treatment of compound I with a base in a suitable solvent followed by the introduction of an appropriate electrophilic derivitizing reagent. From this process compounds of general structure II can be isolated in good yields. Suitable bases include tertiary alkylamines (i.e., triethylamine), pyridine, 4-dimethylaminopyridine, alkali metal carbonate (i.e., $Na_2CO_3$ or $K_2CO_3$) and alkali metal alkoxides (i.e., sodium ethoxide or potassium t-butoxide). Suitable solvents include ethers (i.e., tetrahydrofuran), pyridine, acetone, acetonitrile, alcohols (i.e., methanol, ethanol, isopropanol

and t-butanol) and polar aprotic solvents (i.e., DMSO and DMF). Suitable electrophilic reagents include alkyl halides, arylalkyl halides (i.e., benzyl chloride), carboxylic acid chlorides, alkyl chloro formates, aryl chloro formates, N,N-dialkyl carbamoyl chlorides, alkyl sulfonyl chlorides, aryl sulfonyl chlorides, alkyl chloro thioformates

(i.e., $Cl\overset{\overset{S}{\|}}{C}OR$)

and aryl chlorothioformates

(i.e., $Cl\overset{\overset{S}{\|}}{C}OAr$)

SCHEME IV

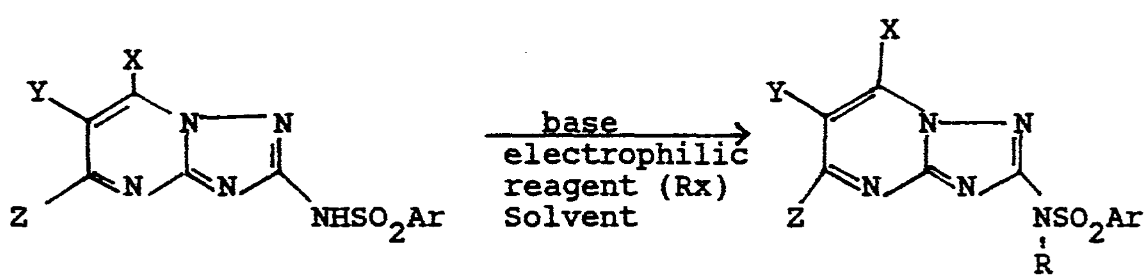


I　　　　II

Compounds of the present invention represented by structure III are also derived from compounds represented by structure I as illustrated in Scheme V. The general process involves the reduction of compounds of general structure I with an appropriate reducing agent in a suitable solvent to yield compounds of general structure III. Reducing agents which are effective include metal

hydrides (i.e., sodium borohydride) in the presence of acids (i.e., methane sulfonic acid) and hydrogen in the presence of normal hydrogenation catalysts (i.e., palladium on carbon). For reductions with metal hydrides polar aprotic solvents (i.e., DMSO) are most frequently used. For reductions using hydrogen and a catalyst, alcohols (i.e., ethanol) are most frequently employed as solvents.

SCHEME V

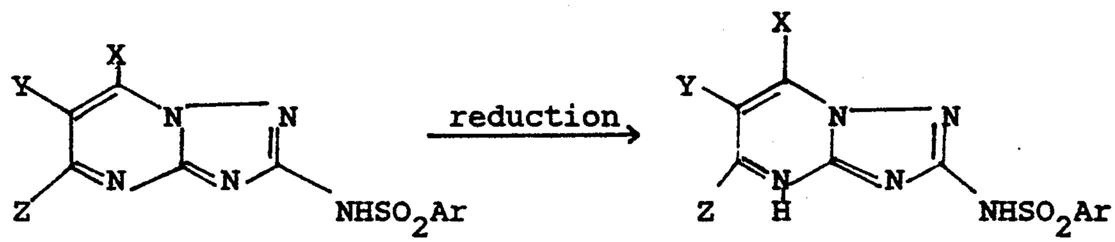


I III

Using the routes illustrated above or minor variations based on the principles illustrated above the novel compounds of this invention can be prepared.

The invention is further illustrated by the following examples.

Example 1A 2-Amino-5,7-dimethyl-1,2,4-triazolo-(1,5-a)pyrimidine

A mixture of 49.5 g (0.500 mol) of 3,5-diamino-1,2,4-triazole, 100 g (1.00 mol) of 2,4-pentanedione, 400 g (1.00 mol) of 10 percent aqueous NaOH in 400 ml of EtOH was heated at reflux for 1.5 hours. After cooling to room temepratuге the solid was collected by filtration and dried in vacuo to afford 45.0 g (55 percent) of pale

yellow solid, m.p. >320°C: IR (KBr) 3320, 3145, 1652, 1560 and 1535 $cm^{-1}$; 'H NMR ($CF_3COOD$) δ11.16 (2H, broad s, $-NH_2$), 7.42 (1H, s, aromatic H), 2.88 (3H, s, $-CH_3$) and 2.80 (3H, s, $-CH_3$).

Analysis:

Calculated for $C_7H_9N_5$: C,51.52; H, 5.56; N, 42.92.

Found: C, 51.12; H, 5.44; N, 42.85.

Example 1B N-(5,7-Dimethyl-1,2,4-triazolo(1,5-a)-pyrimidin-2-yl)-2-thiophene sulfonamide

A mixture of 4.24 g (26.0 mmol) of 2-amino-5,7-dimethyl-1,2,4-triazolo(1,5-a)pyrimidine, 5.08 g (27.8 mmol) of 2-thiophene sulfonyl chloride and 0.16 g (1.3 mmol) of 4-dimethylaminopyridine in 30 ml of dry pyridine was heated at reflux for 47 hours. The majority of the pyridine was removed by evaporation at reduced pressure and the residue was taken up in 1N NaOH, treated with charcoal and filtered through celite. The filtrated was treated with charcoal and filtered through celite. The yellow filtrate was cooled in an ice bath and acidified with concentrated HCl to precipitate a yellow solid. Drying in vacuo gave 0.50 g (6 percent) of the desired sulfonamide as a yellow powder, m.p. 236-237.5°C: IR (KBr) 3390 (broad), 1493, 1375 and 1156 $cm^{-1}$; 'H NMR ($DMSO-d_6$) δ12.3 (1H, broad, $-SO_2NH-$), 7.7-8.1 (2H, m, thiophene H's at 3- and 5-positions), 6.9-7.3 (2H, m including S at 7.03, thiophene H at 4-position and pyrimidine H), 2.62 (3H, s, $-CH_3$) and 2.49 (3H, s, $-CH_3$).

Analysis:

Calculated for $C_{11}H_{11}N_5O_2S_2$: C, 42.71; H,3.58; N, 22.64; S, 20.73.

Found: C, 42.46; H, 3.39; N,22.97; S, 20.48.

Example 2 N-Acetyl-2,6-dichloro-N-(5,7-dimethyl-1,2,4-triazolo[1,5-a]-pyrimidin-2-yl)-benzenesulfonamide

A solution of 0.50 g (1.3 mmol) of 2,6-dichloro--N-(5,7-dimethyl-1,2,4-triazolo[1,5-a]pyrimidin-2-yl)benzenesulfonamide, 2.12 ml (22.5 mmol) of acetic anhydride and 0.18 ml (1.3 mmol) of triethylamine in 4 ml of DMF was heated at 85°C for 3 days. After cooling to room temperature, the product separated from solution and was collected by filtration and dried in vacuo to yield 0.20 g (39 percent) of the desired product as a solid, mp 219-222°C. The product was characterized by IR and NMR spectroscopy.

Analysis:

| | | |
|---|---|---|
| Calculated for $C_{15}H_{13}Cl_2N_5O_3S$ : | | C,43.49; H, 3.16; N, 16.91; Cl, 17.12; S, 7.74. |
| | Found: | C,43.79; H, 3.00; N, 17.03; Cl, 16.98; S, 7.99. |

Example 3 N'-Cyano-N-(2-nitrophenylsulfonyl)-2--methylisothiourea.

A mixture of 2.02 g (10.0 mmol) of 2-nitrobenzene-sulfonamide, 1.46 g (10.0 mmol) of dimethyl N-cyanodithioiminocarbonate and 1.38 g (10.0 mmol) of powdered, anydrous $K_2CO_3$ in 16 ml of acetone was heated at reflux for 20 hours. The reaction mixture was filtered and the solid collected was washed several times with acetone. The filtrate was evaporated and the orange oily residue was triturated with ether to afford a solid. The solid was collected by filtration, washed with ether and

suspended in 10 ml of IN HCl. After stirring for 1 hour the solid was collected by filtration, washed with water and dried to yield 1.65 g (55 percent) of the desired product as a cream colored solid, mp 122°C (decomposition). IR and $^1H$ NMR spectra were consistent with the assigned structure.

Analysis:

Calculated for $C_9H_8N_4O_4S_2$: C, 36.00; H, 2.69; N, 18.66; S, 21.35.

Found: C, 36.10; H, 2.74; N, 18.72; S, 21.22.

Example 4 N-(5-Amino-1,2,4-triazol-3-yl)-2--nitrobenzenesulfonamide

A suspension of 29.4 g (98.0 mmol) of N'-Cyano-N--(2-nitrophenylsulfonyl)-S-methylisothiourea in 100 ml of acetonitrile was treated with 6.2 ml (6.3 g, 0.20 mol) of anhydrous hydrazine. A mild exothermic reaction occurred as the reaction mixture became homogeneous. After stirring for 9 days the precipitated solid was collected by filtration and dried to afford 22.9 g of yellow solid. The crude product was recrystallized from HaAc to yield a total of 15.9 g (57 percent) of the desired product as a pale yellow solid, mp 255-256°C. IR and $^1H$ NMR spectra were consistent with the assigned structure.

Analysis:

Calculated for $C_8H_8N_6O_4S$: C, 33.80; H, 2.84; N, 29.57; S, 11.28.

Found: C, 34.11; H, 2.79; N, 29.35; S, 11.50.

Example 5 N-(5,7-dimethyl-1,2,4-triazolo[1,5-a]-pyrimidin-2-yl)-2-nitrobenzenesulfonamide

A mixture of 2.43 g (9.00 mmol) of N-(5-amino--1,2,4-triazol-3-yl)-2-nitrobenzenesulfonamide and 1.85 ml (1.80 g, 18.0 mmol) of 2,4 pentanedione in 25 ml of glacial acetic acid was heated at reflux for 19 hours. After cooling to room temperature, the solid which separated was collected by filtration, washed with acetic acid and dried in vacuo to yield 2.58 g (82 percent) of the desired product as an off-white crystalline solid, mp 255-256°C. IR and $^1H$ NMR spectra were in agreement with the assigned structure.

Analysis:

Calculated for $C_{13}H_{12}N_6O_4S$: C, 44.83; H, 3.47; N, 24.13; S, 9.20.

Found: C, 44.88; H, 3.34; N, 24.51; S, 9.09.

Example 6 N'-Cyano-N-(2,5-dichlorophenylsulfonyl)--S-methylisothiourea

A solution of 10.6 g (43.2 mmol) of 2,5-dichlorobenzenesulfonamide, 7.15 g (44.0 mmol) of 90 percent dimethyl N-cyanodithioiminocarbonate and 1.8 g (44 mmol) of NaOH in 60 ml of ethanol and 10 ml of $H_2O$ was heated at reflux for 6 hours. After cooling to room temperature the reaction mixture was poured into 600 ml of ice water. The resulting solution was acidified with 6N HCl to separate 2.2 g of the desired product as a white solid. Concentration of the filtrate gave an additional 8.5 g of the desired product. The total yield of material was 10.7 g (76 percent) of white solid, mp 145°C. IR and $^1H$ NMR spectra were consistent with the assigned structure.

Analysis:
Calculated for $C_9H_7Cl_2N_3O_2S_2$: C, 33.34; H, 2.18; N, 12.96.
Found: C, 33.50; H, 2.39; N, 12.82.

Example 7 N-(5-Amino-1,2,4-triazol-3-yl)-2,5--dichlorobenzenesulfonamide

A mixture of 8.51 g (26.2 mmol) of N'-cyano-N--(2,5-dichlorophenylsulfonyl)-S-methylisothiourea and 10 ml (10 g, 0.20 mol) of hydrazine monohydrate in 85 ml of ethanol was heated at reflux for 30 minutes. After cooling to room temperature, the solid which separated was collected and suspended in 170 ml of $H_20$ and the suspension was acidified with concentrated aqueous HCl. After stirring the suspension for 4 hours the solid was collected and dried in vacuo to yield 5.10 g (57 percent) of the desired product as a hydrochloride salt, mp 306-308°C. IR and $^1H$ NMR spectra were consistent with the assigned structure.

Analysis:
Calculated for $C_8H_7Cl_2N_5O_2S \cdot HCl$: C, 27.88; H, 2.34; N, 20.32.
Found: C, 28.36; H, 2.50; N, 19.78.

Example 8 N-(5,7-dimethyl-1,2,4-triazolo[1,5-a]-pyrimidin-2-yl)-2,5-dichlorobenzene--sulfonamide

A solution of 4.60 g (13.3 mmol) of N-(5-amino--1,2,4-triazol-3-yl)-2,5-dichlorobenzenesulfonamide hydrochloride and 4.0 g (40 mmol) of 2,4-pentanedione in 60 ml of glacial acetic acid was heated at reflux for 4

hours. The reaction mixture was cooled to room temperature and poured into 500 ml of ice water to separate a solid. The solid was collected by filtration and dried to yield 4.53 g (92 percent) of the desired product as a white solid, mp 216.5-218.5°C.

Analysis:

Calculated for $C_{13}H_{11}Cl_2N_5O_2S$: C, 41.95; H, 2.98; N, 18.81.

Found: C, 41.83; H, 3.10; N, 18.67.

Example 9 2-Chloro-N-(5-methyl-7-trifluoromethyl--1,2,4-triazolo[1,5-a]pyrimidin-2-yl)--benzenesulfonamide.

A mixture of 2.19 g (8.00 mmol) of N-(5-amino--1,2,4-triazol-3-yl)-2-chlorobenzenesulfonamide and 1.09 ml (1.38 g, 8.96 mmol) of 1,1,1-trifluoro-2,4-pentanedione in 9 ml of glacial acetic acid was heated at reflux for 21 hours. After cooling to room temperature, the reaction mixture was poured into a mixture of ice and water. The solid which separated was collected by filtration, washed with water and dried to yield 2.90 g (93 percent) of the desired product as a white solid, mp 203-204.5°C. IR and $^1H$ NMR spectra were in agreement with the assigned structure.

Analysis:

Calculated for $C_{13}H_9ClF_3N_5O_2S$: C, 39.86; H, 2.32; H, 17.88; Cl, 9.05; S, 8.18.

Found: C, 40.23; H, 2.31; N, 18.22; Cl, 9.13; S, 8.26.

Example 10    2-Chloro-N-(7-methyl-1,2,4-triazolo-[1,5-a]pyrimidin-2-yl)benzenesulfonamide

A sample of 3.0 ml (2.7 g, 20 mmol) of acetylacetaldehyde dimethylacetal was added to a solution of 2.74 g (10.0 mmol) of N-(5-amino-1,2,4-triazol-3-yl)-2--chlorobenzenesulfonamide in 20 ml of glacial acetic acid at reflux over 12 hours. After the addition was complete the reaction mixture was heated at reflux for 15 hours and cooled to room temperature. The solid which separated was collected by filtration, washed with acetic acid and dried to yield 1.92 g (59 percent) of the desired product as white solid, mp 267.5-269°C. IR and $^1H$ NMR spectra were in agreement with the assigned structure.

Analysis:

| | | |
|---|---|---|
| Calculated for $C_{12}H_{10}ClN_5O_2S$: | | C, 44.52; H, 3.11; N, 21.63; Cl, 10.95; S, 9.90. |
| | Found: | C, 44.36; H, 3.07; N, 21.69; Cl, 10.82; S, 10.15. |

Example 11    2-Chloro-N-(1,2,4-triazolo[1,5-a]-pyrimidin-2-yl)benzenesulfonamide

A mixture of 2.74 g (10.0 mmol) of N-(5-amino--1,2,4-triazol-3-yl)-2-chlorobenzenesulfonamide and 3.3 ml (3.3g, 20 mmol) of malonaldehyde bis(dimethylacetal) in 10 ml of glacial acetic acid was heated at reflux for 24 hours. After cooling to room temperature, the solid which separated was collected by filtration, washed with acetic acid and dried to yield 1.78 g (58 percent) of the desired product as tan solid, mp 253.5-256.5°C. IR and $^1H$ NMR spectra were in agreement with the assigned structure.

Analysis:

Calculated for $C_{11}H_8ClN_5O_2S$: C, 42.66; H, 2.60; N, 22.61; Cl, 11.45; S, 10.35.

Found: C, 42.97; H, 2.60; N, 22.42; Cl, 11.19; S, 10.07.

Example 12 2-Chloro-N-(6-Chloro-1,2,4-triazolo-[1,5-a]pyrimidin-2-yl)benzenesulfonamide

A mixture of 2.46 g (9.00 mmol) of N-(5-amino--1,2,4-triazol-3-yl)-2-chlorobenzenesulfonamide and 1.67 g (9.90 mmol) of mucochloric acid in 20 ml of DMF was heated to reflux for 16.5 hours. After cooling to room temperature, the solvent was removed by evaporation at reduced pressure and the residue was treated with 20 ml of 0.5 N NaOH. After stirring vigorously for ~ 30 minutes the mixture was filtered through celite and the filtrate was acidified with 2N HCl. The solid which separated was collected by filtration, washed with water and recrystallized from acetic acid - water to yield 0.70 g (23 percent) of the desired product as a light brown solid, mp 258.5-260.5°C. IR and $^1H$ NMR spectra were in agreement with the assigned structure.

Analysis:

Calculated for $C_{11}H_7Cl_2N_5O_2S$: C, 38.39; H, 2.05; N, 20.35; Cl, 20.60; S, 9.32.

Found: C, 38.74; H, 2.08; N, 20.84; Cl, 19.54; S, 8.70.

Example 13 2-Chloro-N-(6-methyl-1,2,4-triazolo-[1,5-a]pyrimidin-2-yl)benzenesulfonamide

A mixture of 2.02 g (8.36 mmol) of 1,3-bis(dimethylamino)-2-methyltrimethinium perchlorate and 2.29 g (8.36 mmol) of N-(5-amino-1,2,4-triazol-3-yl)-2-chlorobenzenesulfonamide in 25 ml of glacial acetic acid was heated at reflux for 19 hours. The solvent was removed by evaporation at reduced pressure, and the residue was treated with 20 ml of 0.5N NaOH. Some additional $^1$N NaOH was added to dissolve all of the material (~ pH 10). The solution was filtered and the filtrate was acidified with 2N HCl to precipitate a solid. The solid was collected by filtration, washed with water and dried to yield 2.34 g (87 percent) of the desired product as a pale yellow solid, mp 236-239°C. IR and $^1H$ NMR spectra were in agreement with the assigned structure.

Analysis:

| | |
|---|---|
| Calculated for $C_{12}H_{10}ClN_5O_2S$: | C, 44.52; H, 3.11; N, 21.63; Cl, 10.95; S, 9.90. |
| Found: | C, 44.17; H, 3.05; N, 21.93; Cl, 11.01; S, 9.69. |

Example 14 N-(5-Amino-1,2,4-triazol-3-yl)-2,6--dichlorobenzenesulfonamide.

A mixture of 90.1 g (0.398 mol) of 2,6-dichlorobenzenesulfonamide, 64.7 g (0.398 mol) of dimethyl N-cyanodithioiminocarbonate and 58.3 g (0.420 mol) of powdered anhydrous $K_2CO_3$ in 800 ml of THF was heated at reflux for 3 hours. After cooling to 30°C, 25.3 ml (25.6 g, 0.798 mol) of anhydrous hydrazine was added dropwise over 30 minutes. The resulting mixture was stirred for 3 days at ambient temperature and filtered. The solid

0150974

collected was washed with THF, suspended in 400 ml of water and acidified with 180 ml of acetic acid. The resulting mixture was filtered, and the solid collected was washed with water and dried to yield 112 g (91 percent) of the desired product as a white solid, mp 300°C (decomp.) IR and $^1$H NMR spectra were in agreement with the assigned structure.

Analysis:

Calculated for $C_8H_7Cl_2N_5O_2S$: C, 31.18; H, 2.29; N, 22.73; Cl, 23.01; S, 10.40.

Found: C, 31.39; H, 2.26; N, 22.70; Cl, 22.88; S, 10.25.

The compounds prepared employing the above general procedures and the appropriate starting materials are listed in the following Tables I through XIV.

**TABLE I**

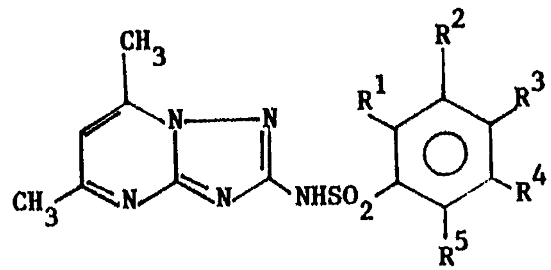

| Compound | $R^1$ | $R^2$ | $R^3$ | $R^4$ | $R^5$ | Melting Point | Elemental Composition | C | H | N | S |
|---|---|---|---|---|---|---|---|---|---|---|---|
| 1 | H | H | H | H | H | 228°-229.5°C | Analysis<br>Calcd. for $C_{13}H_{13}N_5O_2S$:<br>Found: | 51.47<br>51.40 | 4.32<br>4.13 | 23.09<br>23.00 | 10.57<br>10.44 |
| 2 | H | H | $CH_3$ | H | H | 238°-239°C | Analysis<br>Calcd. for $C_{14}H_{15}N_5O_2S$:<br>Found: | 52.98<br>52.28 | 4.76<br>4.64 | 22.07<br>21.80 | |
| 3 | $CF_3$ | H | H | H | H | 246.5-248°C | Analysis<br>Calcd. for $C_{14}H_{12}F_3N_5O_2S$:<br>Found: | 45.28<br>45.31 | 3.26<br>3.33 | 18.86<br>18.74 | 8.63<br>8.71 |

TABLE I (Continued)

| Compound | $R^1$ | $R^2$ | $R^3$ | $R^4$ | $R^5$ | Melting Point | Elemental Composition | | | | | |
|---|---|---|---|---|---|---|---|---|---|---|---|---|
| 4 | $CH_3$ | H | H | H | H | 202.5°-203.5°C | Analysis | C | H | N | S | |
| | | | | | | | Calcd. for $C_{14}H_{15}N_5O_2S$: | 52.98 | 4.76 | 22.07 | 10.10 | |
| | | | | | | | Found: | 52.43 | 4.56 | 21.78 | 9.59 | |
| 5 | $NO_2$ | H | H | H | H | 255°-256°C (decomp.) | Analysis | C | H | N | S | |
| | | | | | | | Calcd. for $C_{13}H_{12}N_6O_4S$: | 44.83 | 3.47 | 24.13 | 9.20 | |
| | | | | | | | Found: | 44.88 | 3.34 | 24.51 | 9.09 | |
| 6 | Cl | H | H | H | H | 216.5°-219.5°C | Analysis | C | H | N | Cl | S |
| | | | | | | | Calcd. for $C_{13}H_{12}ClN_5O_2S$: | 46.23 | 3.58 | 20.73 | 10.50 | 9.49 |
| | | | | | | | Found: | 45.97 | 3.66 | 21.01 | 10.73 | 9.30 |
| 7 | H | H | Cl | H | H | 254°-255.5°C | Analysis | C | H | N | | |
| | | | | | | | Calcd. for $C_{13}H_{12}ClN_5O_2S$: | 46.22 | 3.58 | 20.73 | | |
| | | | | | | | Found: | 46.10 | 3.75 | 20.69 | | |
| 8 | H | H | $OCH_3$ | H | H | 198.5°-199.5°C | Analysis | C | H | N | | |
| | | | | | | | Calcd. for $C_{14}H_{15}N_5O_3S$: | 50.44 | 4.54 | 21.01 | | |
| | | | | | | | Found: | 50.43 | 4.70 | 20.99 | | |

TABLE I (Continued)

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 9 | i-Pr | H | i-Pr | H | i-Pr | 283°C (decomp.) | Analysis | | | | | |
| | | | | | | | Calcd. for $C_{22}H_{31}N_5O_2S$: | 61.51 | 7.27 | 16.30 | | |
| | | | | | | | Found: | 61.30 | 7.33 | 16.28 | | |
| 10 | Cl | H | H | H | Cl | 259-261°C | Analysis | | | | | |
| | | | | | | | Calcd. for $C_{13}H_{11}Cl_2N_5O_2S$: | 41.95 | 2.98 | 18.81 | 19.05 | 8.61 |
| | | | | | | | Found: | 41.86 | 2.94 | 19.09 | 18.92 | 8.39 |
| 11 | F | H | H | H | F | 261°-262°C | Analysis | | | | | |
| | | | | | | | Calcd. for $C_{13}H_{11}F_2N_5O_2S$: | 46.02 | 3.27 | 20.64 | | |
| | | | | | | | Found: | 45.94 | 3.19 | 20.79 | | |
| 12 | Cl | Cl | H | H | H | 231-233°C | Analysis | | | | | |
| | | | | | | | Calcd. for $C_{11}H_7Cl_2N_5O_2S$: | 41.95 | 2.98 | 18.81 | | |
| | | | | | | | Found: | 41.83 | 2.90 | 19.55 | | |

TABLE II

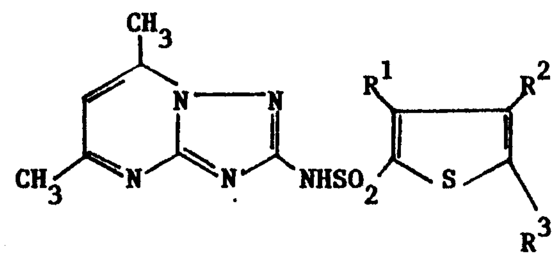

| Compound | $R^1$ | $R^2$ | $R^3$ | Melting Point | Elemental Analysis | | | | | |
|---|---|---|---|---|---|---|---|---|---|---|
| 13 | H | H | H | 236°-237.5°C | Analysis | C | H | N | S | |
| | | | | | Calcd. for $C_{11}H_{11}N_5O_2S_2$: | 42.71 | 3.58 | 22.64 | 20.73 | |
| | | | | | Found: | 42.46 | 3.39 | 22.97 | 20.48 | |
| 14 | H | H | Cl | 200°-202°C | Analysis | C | H | N | Cl | S |
| | | | | | Calcd. for $C_{11}H_{10}ClN_5O_2S_2$: | 38.43 | 2.93 | 20.37 | 10.31 | 18.65 |
| | | | | | Found: | 38.08 | 2.83 | 20.47 | 8.59 | 18.91 |
| 15 | H | Br | Br | 183°-185°C | Analysis | C | H | N | Br | S |
| | | | | | Calcd. for $C_{11}H_9Br_2N_5O_2S_2$: | 28.28 | 1.94 | 14.99 | 34.21 | 13.73 |
| | | | | | Found: | 28.06 | 1.78 | 14.97 | 33.60 | 13.90 |

TABLE III

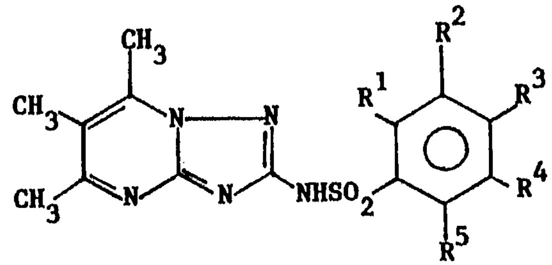

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 16 | Cl | H | H | H | H | 273°-280°C | Analysis Calcd. for $C_{14}H_{14}ClN_5O_2S$: | 47.80 | 4.01 | 19.91 | 10.08 | 9.11 |
| | | | | | | | Found: | 47.78 | 3.90 | 20.19 | 10.20 | 9.17 |
| 17 | Cl | H | H | H | Cl | 347°C | Analysis Calcd. for $C_{14}H_{13}Cl_2N_5O_2S$: | 43.53 | 3.39 | 18.13 | 18.35 | 8.30 |
| | | | | | | | Found: | 43.52 | 3.29 | 18.42 | 18.37 | 8.29 |

TABLE IV

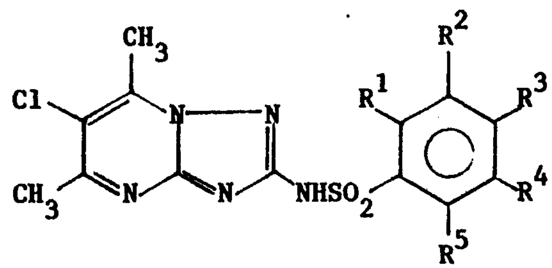

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 18 | Cl | H | H | H | H | 266°-269°C | Analysis | C | H | N | Cl | S |
| | | | | | | | Calcd. for $C_{13}H_{11}Cl_2N_5O_2S$: | 41.95 | 2.98 | 18.81 | 19.05 | 8.61 |
| | | | | | | | Found: | 42.51 | 3.03 | 18.98 | 18.16 | 8.41 |
| 19 | Cl | H | H | H | Cl | 296-297°C | Analysis | C | H | N | | |
| | | | | | | | Calcd. for $C_{13}H_{10}Cl_2N_5O_2S$: | 38.39 | 2.48 | 17.22 | | |
| | | | | | | | Found: | 38.48 | 2.44 | 17.58 | | |

**TABLE V**

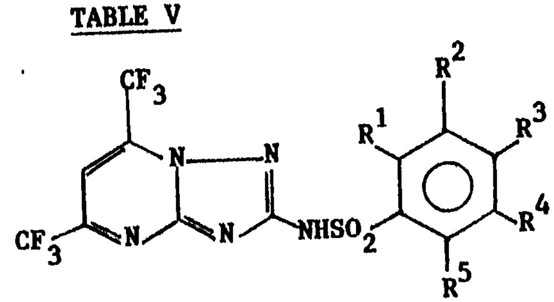

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 20 | Cl | H | H | H | H | 224°-226°C | Analysis<br>Calcd. for $C_{13}H_6ClF_6N_5O_2S$:<br>Found: | 35.02<br>35.31 | 1.36<br>1.38 | 15.71<br>15.95 | 7.95<br>7.72 | 7.19<br>7.40 |
| 21 | Cl | H | H | H | Cl | 238°-240°C | Analysis<br>Calcd. for $C_{13}H_5Cl_2F_6N_5O_2S$:<br>Found: | 32.51<br>32.19 | 2.05<br>1.01 | 20.35<br>14.57 | 14.77<br>14.51 | 6.68<br>6.86 |

**TABLE VI**

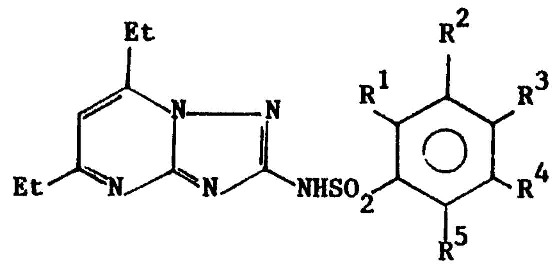

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 22 | Cl | H | H | H | H | 216°-218°C | Analysis | C | H | N | Cl | S |
| | | | | | | | Calcd. for $C_{15}H_{16}ClN_5O_2S$: | 49.25 | 4.41 | 19.14 | 9.69 | 8.76 |
| | | | | | | | Found: | 49.18 | 4.36 | 19.45 | 9.64 | 8.75 |
| 23 | Cl | H | H | H | Cl | 259°-261°C | Analysis | C | H | N | Cl | S |
| | | | | | | | Calcd. for $C_{15}H_{15}Cl_2N_5O_2S$: | 45.01 | 3.78 | 17.50 | 17.72 | 8.01 |
| | | | | | | | Found: | 44.44 | 3.72 | 17.79 | 17.33 | 8.32 |

TABLE VII

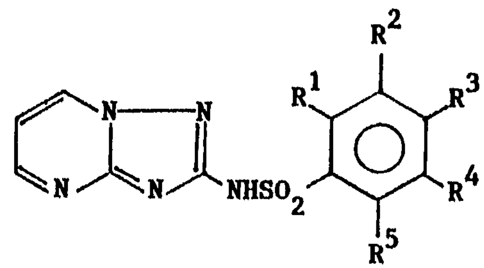

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 24 | Cl | H | H | H | H | 253.5-256.5°C | Analysis | | | | | |
| | | | | | | | Calcd. for $C_{11}H_8ClN_5O_2S$: | 42.66 | 2.60 | 22.61 | 11.45 | 10.35 |
| | | | | | | | Found: | 42.97 | 2.60 | 22.42 | 11.19 | 10.07 |
| 25 | Cl | H | H | H | Cl | 264°-269°C | Analysis | | | | | |
| | | | | | | | Calcd. for $C_{11}H_7Cl_2N_5O_2S$: | 38.38 | 2.05 | 20.35 | 20.61 | 9.32 |
| | | | | | | | Found: | 38.29 | 2.05 | 20.08 | 19.80 | 9.13 |

TABLE VIII

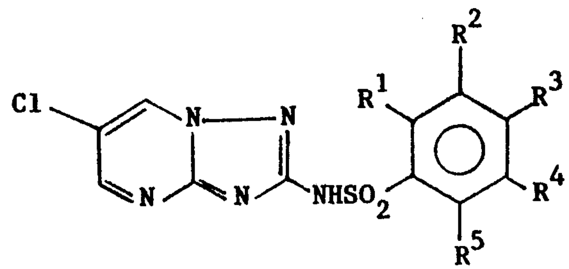

| Compound | $R^1$ | $R^2$ | $R^3$ | $R^4$ | $R^5$ | Melting Point | Elemental Analysis | C | H | N | Cl | S |
|---|---|---|---|---|---|---|---|---|---|---|---|---|
| 26 | Cl | H | H | H | H | 258.5-260.5°C | Analysis | C | H | N | Cl | S |
| | | | | | | | Calcd. for $C_{11}H_7Cl_2N_5O_2S$: | 38.39 | 2.05 | 20.35 | 20.60 | 9.32 |
| | | | | | | | Found: | 38.74 | 2.08 | 20.84 | 19.54 | 8.70 |
| 27 | Cl | H | H | H | Cl | 262°-264°C | Analysis | C | H | N | | |
| | | | | | | | Calcd. for $C_{11}H_6Cl_3N_5O_2S$: | 34.89 | 1.60 | 18.50 | | |
| | | | | | | | Found: | 34.72 | 1.78 | 19.12 | | |

**TABLE IX**

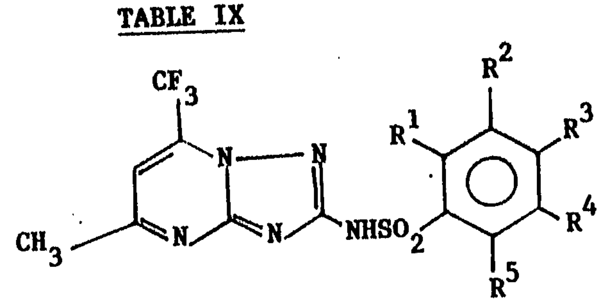

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 28 | Cl | H | H | H | H | 203°-204.5°C | Analysis | | | | | |
| | | | | | | | Calcd. for $C_{13}H_9ClF_3N_5O_2S$: | 39.86 | 2.32 | 17.88 | 9.05 | 8.18 |
| | | | | | | | Found: | 40.23 | 2.31 | 18.22 | 9.13 | 8.26 |

## TABLE X

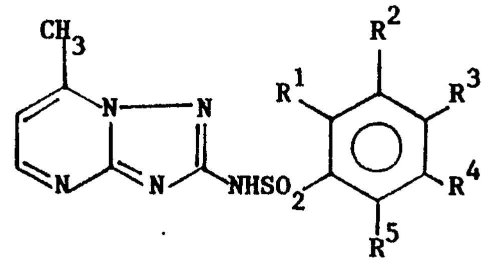

| Compound | $R^1$ | $R^2$ | $R^3$ | $R^4$ | $R^5$ | Melting Point | Elemental Composition | C | H | N | Cl | S |
|---|---|---|---|---|---|---|---|---|---|---|---|---|
| 29 | Cl | H | H | H | H | 267.5°-269°C | Analysis | | | | | |
| | | | | | | | Calcd. for $C_{12}H_{10}ClN_5O_2S$: | 44.52 | 3.11 | 21.63 | 10.95 | 9.90 |
| | | | | | | | Found: | 44.36 | 3.07 | 21.69 | 10.82 | 10.15 |

## TABLE XI

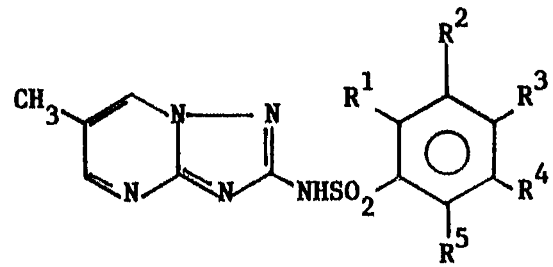

| Compound | $R^1$ | $R^2$ | $R^3$ | $R^4$ | $R^5$ | Melting Point | Elemental Composition | | | | | |
|---|---|---|---|---|---|---|---|---|---|---|---|---|
| | | | | | | | Analysis | C | H | N | Cl | S |
| 30 | Cl | H | H | H | H | 236°-239°C | Calcd. for $C_{12}H_{10}ClN_5O_2S$: | 44.52 | 3.11 | 21.63 | 10.95 | 9.90 |
| | | | | | | | Found: | 44.17 | 3.05 | 21.93 | 11.01 | 9.69 |

TABLE XII

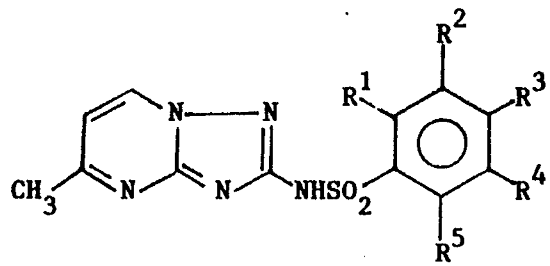

| Compound | $R^1$ | $R^2$ | $R^3$ | $R^4$ | $R^5$ | Melting Point | Elemental Composition | | | |
|---|---|---|---|---|---|---|---|---|---|---|
| | | | | | | | Analysis | C | H | N |
| 31 | Cl | H | H | H | Cl | 305°-310°C (decomp.) | Calcd. for $C_{12}H_9Cl_2N_5O_2S$: | 40.23 | 2.53 | 19.55 |
| | | | | | | | Found: | 40.31 | 2.57 | 20.88 |

TABLE XIII

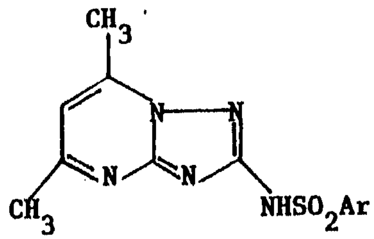

| Compound | Ar | Melting Point | Elemental composition | C | H | N | S |
|---|---|---|---|---|---|---|---|
| 32 | 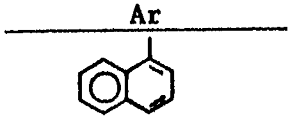 | 221-224°C | Analysis<br>Calcd. for $C_{17}H_{15}N_5O_2S$:<br>Found: | 57.78<br>58.15 | 4.28<br>4.18 | 19.82<br>19.69 | 9.07<br>9.23 |
| 33 | 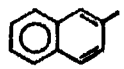 | 242-245°C | Analysis<br>Calcd. for $C_{17}H_{15}N_5O_2S$:<br>Found: | 57.78<br>57.36 | 4.28<br>4.29 | 19.82<br>20.27 | 9.07<br>9.40 |

TABLE XIV

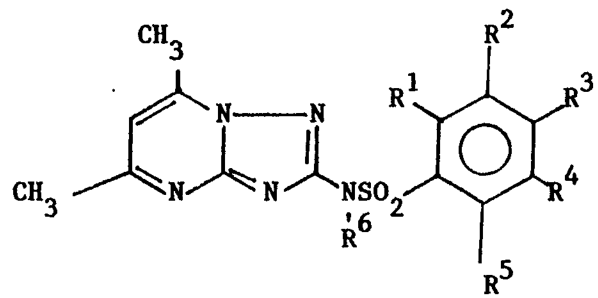

| Compound | $R^1$ | $R^2$ | $R^3$ | $R^4$ | $R^5$ | $R^6$ | Melting Point | Elemental Composition | | | | | |
|---|---|---|---|---|---|---|---|---|---|---|---|---|---|
| 34 | Cl | H | H | H | H | $CH_2Ph$ | 240°-242°C | Analysis | C | H | N | Cl | S |
| | | | | | | | | Calcd. for $C_{20}H_{18}ClN_5O_2S$: | 56.14 | 4.24 | 16.37 | 8.29 | 7.49 |
| | | | | | | | | Found: | 55.62 | 4.08 | 16.52 | 9.51 | 6.72 |
| 35 | H | H | $CH_3$ | H | H | $CH_2Ph$ | 137°-138°C | Analysis | C | H | N | S | |
| | | | | | | | | Calcd. for $C_{21}H_{21}N_5O_2S$: | 61.90 | 5.19 | 17.18 | 7.85 | |
| | | | | | | | | Found: | 61.84 | 5.07 | 17.19 | 7.78 | |
| 36 | Cl | H | H | H | Cl | $COCH_3$ | 219°-222°C | Analysis | C | H | N | Cl | S |
| | | | | | | | | Calcd. for $C_{15}H_{13}Cl_2N_5O_3S$: | 43.49 | 3.16 | 16.91 | 17.12 | 7.74 |
| | | | | | | | | Found: | 43.79 | 3.00 | 17.03 | 16.98 | 7.99 |

The compounds of the present invention are highly effective herbicides. They have utility for broadspectrum pre- and/or postemergence weed control in areas where complete vegetation control is desired. The subject compounds are also useful for selective pre- and/or postemergence weed control in crops such as wheat. Certain of these compounds are effective for the control of nutsedge (cyperus spp.) and some compounds may be used for selective weed control in corn, soybeans and rice.

For all such uses, unmodified active ingredients of the present invention can be employed. However, the present invention embraces the use of a herbicidally-effective amount of the active ingredients in composition form with an inert material known in the art as an agricultural adjuvant or carrier in solid or liquid form. Such adjuvants or carriers must not be phytotoxic to valuable crops particularly at the concentration employed in applying the composition in attempting selective weed control in the presence of crops. If weed control is desired in the absence of crops, it is generally sufficient to employ adjuvants or carriers which do not leave a persistent phytotoxic residue.

Thus, for example, an active ingredient can be dispersed on a finely-divided solid and employed therein as a dust. Also, the active ingredients, as liquid concentrates or solid compositions comprising one or more of the active ingredients can be dispersed in water, typically with aid of a wetting agent, and the resulting aqueous dispersion employed as a spray. In other procedures the active ingredients can be

employed as a constituent of organic liquid compositions, oil-in-water and water-in-oil emulsions or dispersions, with or without the addition of wetting, dispersing, or emulsifying agents.

Suitable adjuvants of the foregoing type are well known to those skilled in the art. The methods of applying the solid or liquid herbicidal formulations similarly are well known to the skilled artisan.

Organic solvents that can be employed include toluene, xylene, kerosene, diesel fuel, fuel oil, and petroleum naphtha, ketones such as acetone, methylethyl ketone and cyclohexanone, chlorinated hydrocarbons such as trichloroethylene, and perchloroethylene, esters such as ethyl acetate, amyl acetate and butyl acetate, ethers, e.g., ethylene glycol monomethyl ether and diethylene glycol monomethyl ether, alcohols, e.g., methanol, ethanol, isopropanol, amyl alcohol, ethylene glycol, propylene glycol, butylcarbitol acetate and glycerine. Mixtures of water and organic solvents, either as emulsions or solutions, can be employed.

The active ingredients of the present invention can also be applied as aerosols, e.g., by dispersing them by means of a compressed gas such as one of the fluorocarbons or one of its hydrocarbon successors.

The active ingredients of the present invention can also be applied with solid adjuvants or carriers such as talc, pyrophyllite, synthetic fine silica, attapulgus clay, kieselguhr, chalk, diatomaceous earth, lime, calcium carbonate, bentonite,

Fuller's earth, cotton seed hulls, wheat flour, soybean flour, pumice, tripoli, wood flour, walnut shell flour, redwood flour and lignin.

As stated, it is frequently desirable to incorporate a surface-active agent in the compositions of the present invention. Such surface-active or wetting agents are advantageously employed in both the solid and liquid compositions. The surface-active agent can be anionic, cationic or nonionic in character.

Typical classes of surface-active agents include alkyl sulfonate salts, alkylaryl sulfonate salts, alkylaryl polyether alcohols, fatty acid esters of polyhydric alcohols and the alkylene oxide addition products of such esters, and addition products of long-chain mercaptans and alkylene oxides. Typical examples of such surface-active agents include the sodium alkylbenzene sulfonates having 10 to 18 carbon atoms in the alkyl group, alkyl phenol ethylene oxide condensation products, e.g., p-isooctylphenol condensed with 20 ethylene oxide units, soaps, e.g., sodium stearate and potassium oleate, sodium salt of propyl-naphthalene sulfonic acid, di(2-ethylhexyl)ester of sodium sulfosuccinic acid, sodium lauryl sulfate, sodium decyl sulfonate, sodium salt of the sulfonated monoglyceride of coconut fatty acids, sorbitan sesquioleate, lauryl trimethyl ammonium chloride, octadecyl trimethyl ammonium chloride, polyethylene glycol lauryl ether, polyethylene glycol esters of fatty acids and rosin acids, e.g., Ethofat 7 and 13, sodium N-methyl-N-oleyl taurate, sodium dibutylnaphthalene sulfonate, sodium lignin sulfonate, polyethylene glycol stearate, sodium dodecyl benzene sulfonate, tertiary dodecyl

polyethylene glycol thioether (nonionic 218), long-chain ethylene oxide-propylene oxide condensation products e.g., Pluronic 61 (molecular weight about 1000), polyethylene glycol ester of tall oil acids, sodium octophenoxyethoxyethyl sulfate, tris(polyoxyethylene)-sorbitan monostearate (Tween 60), and sodium dihexyl-sulfosuccinate.

The herbicidally effective concentration of the active ingredients in solid or liquid compositions generally is from about 0.0003 to about 95 percent by weight or more. Concentrations from about 0.05 to about 50 percent by weight are often employed. In compositions to be employed as concentrates, the active ingredient can be present in a concentration from about 5 to about 98 weight percent, preferably 15-50 weight percent. The active ingredient compositions can also contain other compatible additaments, for example, phytotoxicants, plant growth regulants, pesticides and the like and can be formulated with solid particulate fertilizer carriers such as ammonium nitrate, urea and the like.

In further embodiments, the compounds of the present invention or compositions containing the same, can be advantageously employed in combination with one or more additional pesticidal compounds. Such additional pesticidal compounds may be insecticides, nematocides, miticides, arthropodicides, herbicides, fungicides or bactericides that are compatible with the compounds of the present invention in the medium selected for application. In such embodiments, the pesticidal compound is employed as a supplemental toxicant for the same or for a different pesticidal use or as an additament.

The compounds of the present invention are particularly useful in combination with other herbicides including the substituted urea herbicides such as 3-(3,4-dichlorophenyl)-1,1-dimethylurea, 3-(3,4-dichlorophenyl)-1-methoxy-1-methylurea (Lorox®) and 1,1-dimethyl-3-(α,α,α-trifluoro-m-tolyl)urea (Cotoran®); the triazines such as 2-chloro-4-(ethylamino)-6-(isopropylamino)-s-triazine and 2-chloro-4-(1-cyano-1-methylethylamino)-6-ethylamino-s-triazine (Bladex®); the uracils such as 5-bromo-3-sec-butyl-6-methyluracil; N-(phosphonomethyl)glycine; the phenoxys such as 2,4-dichlorophenoxyacetic acid; picolinic acids such as 4-amino-3,5,6-trichloropicolinic acid (Tordon®) and 3,6-dichloropicolinic acid (Lontrel®); 4-chloro-2-butynyl-3-chlorophenyl carbamate (Carbyne®); diisopropylthiocarbamic acid, ester with 2,3-dichloroallyl alcohol (Avadex®); diisopropylthiocarbamic acid, ester with 2,3,3-trichloroallyl alcohol (Avadex® BVD); ethyl-N-benzoyl-N-(3,4-dichlorophenyl)-2-aminopropionate (Suffix®); 1,2-dimethyl-3,5-diphenylpyrazolium methylsulfate (Avenge®); methyl (2-[4-(2,4-dichlorophenoxy)-phenoxy]propanoate) (Hoelon®); butyl 2-[4-[(5-(trifluoromethyl)-2-pyridinyl)oxy]phenoxy]propanoate (Fusilade®); esters of 2-[4-[(3-chloro-5-trifluoromethyl)-2-pyridinyl)oxy]phenoxy]propionic acid; 4-amino-6-tert-butyl-3-(methylthio)-1,2,4-triazin-5-(4H)-one (Lexone®); 3-isopropyl-1H-2,1,3-benzothiadiazin-(4)-3H-one 2,2-dioxide; α,α,α-trifluoro-2,6-dinitro-N,N-dipropyl-p-toluidine; 1,1'-dimethyl-4,4'-bipyridinium ion; 2-chloro-2',6'-diethyl-(methoxymethyl)acetanilide; and 2-[1-(ethoxyimino)-butyl]-5-[(2-ethylthio)propyl]-3-hydroxy-2-cyclohexen-1-one (Poast®).

The rates of application for compounds of the invention are determined by a number of factors including the active ingredient being applied, the particular action desired (e.g., general or selective control), the plant species to be modified and the stage of growth thereof, the part of the plant to be contacted with the toxic active ingredient, the formulation selected, weather and climate, etc. Thus, it is to be understood that all of the active ingredients of the present invention and compositions containing the same may not be equally effective at similar concentrations or against the same plant species. In non-selective preemergence and foliar treatments, the active ingredients of the invention are usually applied at an approximate rate of from about 0.1 to about 10 pounds/acre. In pre- and postemergence operations for selective uses, a dosage of about 0.01 to about 10 pounds/acre is generally applicable, a rate of 0.01 to 4 pounds/acre being preferred.

Plant species in the following tests were the following:

| | Common Name | Scientific Name |
|---|---|---|
| A. | cotton | *Gossypium spp.* |
| B. | rape | *Brassica napus* |
| C. | soybean | *Glycine max.* |
| D. | sugar beet | *Beta saccharifera* |
| E. | cocklebur | *Xanthium spp.* |
| F. | jimsonweed | *Datura stramonium* |
| G. | annual morning glory | *Ipomoea spp.* |
| H. | pigweed | *Amaranthus spp.* |
| I. | velvetleaf | *Abutilon theophrasti* |

(Continued)

| | Common Name | Scientific Name |
|---|---|---|
| J. | corn | Zea mays |
| K. | rice | Oryza sativa |
| L. | sorghum | Sorghum vulgare |
| M. | wheat | Triticum aestivum |
| N. | barnyardgrass (watergrass) | Echinochloa crusgalli |
| O. | crabgrass | Digitaria spp. |
| P. | yellow foxtail | Setaria lutescens |
| Q. | johnson grass | Sorghum halepense |
| R. | wild oats | Avena fatua |
| S. | sprangletop | Leptochloa filiformis |
| T. | yellow nutsedge | Cyperus esculentus |

In representative operations, each compound to be utilized in a series of tests is dissolved in acetone to one-half of the final volume (twice the final concentration) to be used and the acetone solution in each case is admixed with an equal volume of water containing 0.1 percent by weight of surface active material. The compositions, generally in the nature of an emulsion, were employed to spray separate respective plant species which had been grown to a 2-4 leaf stage in soil of good nutrient content in a greenhouse. Sufficient amounts were employed to provide various application rates as listed in the table. The various beds were positioned side by side and exposed to substantially identical conditions of temperature and light. Each bed was maintained so as to prevent any interaction with test compounds in different seed beds. Other plants were left untreated to serve as controls. After treatment, the

plants were maintained for about two weeks under greenhouse conditions conducive for good plant growth and watered as necessary. The specific plant species, test compound and dosage and the percent postemergent control obtained are set forth in the table below. Control refers to the reduction in growth compared to the observed results of the same untreated species.

TABLE XV

POSTEMERGENT CONTROL OF PLANT SPECIES

| Compound | Dosage (ppm) | Plant Species A | B | C | D | E | F | G | H | I | J | K | L | M | N | O | P | Q | R | T |
|---|---|---|---|---|---|---|---|---|---|---|---|---|---|---|---|---|---|---|---|---|
| 1 | 750 | 40 | 90 | 80 | 100 | 80 | 10 | 0 | - | 80 | 0 | - | 0 | 0 | 0 | - | - | 0 | 0 | 0 |
| 2 | 700 | 0 | 0 | 0 | 40 | 0 | 0 | 20 | - | 0 | 0 | - | 0 | 0 | 0 | - | - | 0 | 0 | 0 |
| 3 | 500 | 50 | 90 | 35 | 95 | 85 | 90 | 15 | 100 | 95 | 0 | 0 | 80 | 0 | 0 | 0 | 0 | 10 | 0 | 0 |
| 4 | 2000 | 40 | 60 | 50 | 75 | 30 | - | 0 | 98 | 70 | 0 | 25 | 0 | 0 | 0 | 0 | 0 | 10 | 0 | 0 |
| 5 | 500 | 0 | - | 35 | 50 | 0 | - | 0 | 80 | 75 | 0 | 0 | 10 | 0 | 0 | 0 | 0 | 0 | 0 | 0 |
| 6 | 500 | 100 | 90 | 85 | 100 | 80 | 80 | 75 | 100 | 100 | 50 | 0 | 60 | 0 | 90 | 0 | 0 | 20 | 0 | 10 |
| 10 | 250 | 70 | 80 | 60 | 100 | 50 | - | 50 | 100 | 70 | 0 | 30 | 50 | 0 | 30 | 20 | 60 | 0 | 0 | 40 |
| 11 | 500 | 0 | 80 | 0 | 0 | 0 | 60 | 0 | 80 | 0 | 0 | 0 | - | 0 | 0 | 0 | 0 | 0 | 0 | 0 |
| 13 | 1000 | 40 | 90 | 100 | 100 | 100 | 100 | 10 | 100 | 85 | 0 | 70 | 15 | 0 | 0 | 0 | 0 | 40 | 10 | 0 |
| 16 | 2000 | 0 | - | 10 | 10 | 10 | - | 10 | 80 | 0 | 0 | 0 | 0 | 0 | 0 | 0 | 0 | 20 | 0 | 0 |
| 17 | 2000 | 20 | 0 | 25 | 20 | 0 | 0 | 0 | 40 | - | 0 | 20 | 0 | 0 | 0 | 0 | 0 | 0 | 0 | 0 |
| 18 | 2000 | 40 | - | 40 | 10 | 0 | - | 0 | 100 | 90 | 0 | 0 | 0 | 0 | 0 | 0 | 0 | 0 | 0 | 0 |

**TABLE XV (continued)**

**POSTEMERGENT CONTROL OF PLANT SPECIES**

| Compound | Dosage (ppm) | Plant Species A | B | C | D | E | F | G | H | I | J | K | L | M | N | O | P | Q | R | T |
|---|---|---|---|---|---|---|---|---|---|---|---|---|---|---|---|---|---|---|---|---|
| 19 | 4000 | 10 | - | - | - | - | - | 20 | 40 | 50 | - | - | - | - | 0 | 0 | 30 | - | 0 | 0 |
| 20 | 2000 | 0 | - | 20 | 0 | 30 | 20 | 10 | 50 | 0 | 0 | 0 | - | 0 | 0 | 10 | 0 | 10 | 0 | 0 |
| 21 | 2000 | 0 | 75 | 0 | 0 | 0 | 60 | 30 | 0 | 0 | 0 | 0 | 0 | 0 | 0 | 0 | 0 | 0 | 0 | 0 |
| 22 | 2000 | 50 | 85 | 50 | 35 | 40 | 80 | 0 | 100 | 75 | 0 | 0 | 0 | 0 | 15 | 0 | 0 | 0 | 0 | 0 |
| 23 | 2000 | 0 | 20 | 30 | 0 | 0 | - | 0 | 80 | - | 0 | 0 | 0 | 0 | 0 | 50 | 0 | 0 | 0 | 0 |
| 24 | 4000 | 0 | - | - | - | - | - | 20 | 40 | 20 | - | - | - | - | 0 | 0 | 0 | - | 0 | 0 |
| 25 | 2000 | 20 | 70 | 75 | 25 | 0 | 0 | 0 | 50 | 100 | 0 | 0 | 20 | 0 | 0 | 60 | 0 | 0 | 0 | 0 |
| 26 | 2000 | 0 | - | 40 | 0 | 0 | - | 0 | 40 | 15 | 0 | 10 | 0 | 0 | 0 | 0 | 60 | 0 | 0 | 0 |
| 28 | 2000 | 50 | - | 10 | 20 | 0 | - | 0 | 95 | 20 | 0 | 0 | 0 | - | 0 | 0 | 0 | 0 | 0 | 0 |
| 29 | 4000 | 40 | - | - | - | - | - | 50 | 50 | 98 | - | - | - | - | 0 | 0 | 0 | - | 0 | 0 |
| 30 | 2000 | 30 | 100 | 20 | 0 | 15 | - | 0 | 90 | 45 | 0 | 0 | 0 | 0 | 0 | 0 | 0 | 0 | 0 | 0 |
| 33 | 2000 | 0 | 70 | 40 | 50 | - | 0 | 0 | 35 | 0 | 0 | 0 | 0 | 0 | 0 | 0 | 0 | 0 | 0 | 0 |

So as to clearly illustrate the phytotoxic properties of the various active ingredients of the present invention applied preemergently, a controlled greenhouse experiment is described below.

The seeds of various species of plants were planted in beds of good agricultural soil in a greenhouse. A number of compositions of the present invention, generally in the nature of an aqueous emulsion, were applied at rates listed in the table so as to deposit a predetermined amount of active ingredients uniformly throughout the surface of the bed. Another seed bed was treated only with water to serve as a control. After treatment the seed beds were maintained for two weeks under greenhouse conditions conducive for good plant growth and watered as necessary. The specific plant species, test compound, and dosage and the percent preemergent control are set forth in the table below. Control refers to the reduction in growth compared to the observed results of the same untreated species.

## TABLE XVI

## PREEMERGENT CONTROL OF PLANT SPECIES

| Compound | Dosage (kg/ha) | Plant Species A | B | C | D | F | G | H | I | J | K | L | M | N | O | Q | R | P | T |
|---|---|---|---|---|---|---|---|---|---|---|---|---|---|---|---|---|---|---|---|
| 1 | 2.24 | 80 | 70 | 80 | 80 | 0 | 30 | 70 | 70 | 0 | 50 | 0 | 0 | 0 | 0 | 0 | 40 | 0 | 30 |
| 3 | 1.12 | 100 | 100 | 80 | 100 | 30 | 60 | - | 100 | 0 | 40 | 40 | 0 | 0 | - | 10 | 0 | 0 | 70 |
| 4 | 11.2 | 80 | - | - | - | - | 90 | 90 | - | - | - | - | - | 0 | 0 | - | 0 | 0 | - |
| 5 | 11.2 | 60 | - | - | - | - | 80 | 98 | 90 | - | - | - | - | 40 | 20 | - | 20 | 30 | - |
| 6 | 0.28 | 40 | 100 | 60 | 70 | 30 | 0 | 0 | 0 | 0 | 0 | 0 | 0 | 0 | 0 | 0 | 100 | 0 | 0 |
| 10 | 4.48 | 95 | 100 | 85 | 100 | 95 | 80 | - | 100 | 30 | 100 | 0 | 0 | 70 | - | 20 | 0 | 40 | 100 |
| 11 | 2.24 | 80 | 100 | 60 | 90 | 100 | 40 | - | 0 | 60 | 50 | 30 | 50 | 50 | - | 30 | 60 | 30 | 0 |
| 13 | 1.12 | 60 | 90 | 50 | 100 | - | 40 | 98 | 80 | 20 | 20 | 30 | 30 | 0 | 0 | 0 | 0 | 0 | 90 |

TABLE XVI (continued)

PREEMERGENT CONTROL OF PLANT SPECIES

| Compound | Dosage (kg/ha) | Plant Species A | B | C | D | F | G | H | I | J | K | L | M | N | O | Q | R | P | T |
|---|---|---|---|---|---|---|---|---|---|---|---|---|---|---|---|---|---|---|---|
| 14 | 4.48 | 0 | 90 | 40 | 40 | 0 | 0 | - | 20 | 0 | 0 | 0 | 0 | 0 | - | 0 | 0 | 0 | 0 |
| 17 | 11.2 | 20 | - | - | - | - | 60 | - | 80 | - | - | - | - | 0 | 0 | - | 0 | 0 | - |
| 18 | 11.2 | 50 | - | - | - | - | 70 | 98 | 70 | - | - | - | - | 0 | 0 | - | 0 | 0 | - |
| 19 | 11.2 | - | - | - | - | - | 90 | 98 | 100 | - | - | - | - | 0 | 0 | - | 0 | 0 | - |
| 22 | 11.2 | 50 | - | - | - | - | 98 | 90 | 98 | - | - | - | - | 0 | 0 | - | 0 | 0 | - |
| 24 | 11.2 | 40 | - | - | - | - | 60 | 100 | 98 | - | - | - | - | 0 | 0 | - | 0 | 0 | - |
| 25 | 11.2 | 80 | - | - | - | - | 40 | 98 | - | - | - | - | - | 0 | 0 | - | 0 | 0 | - |

0150974

TABLE XVI (continued)

PREEMERGENT CONTROL OF PLANT SPECIES

| Compound | Dosage (kg/ha) | Plant Species | | | | | | | | | | | | | | | | | |
|---|---|---|---|---|---|---|---|---|---|---|---|---|---|---|---|---|---|---|---|
| | | A | B | C | D | F | G | H | I | J | K | L | M | N | O | Q | R | P | T |
| 26 | 4.48 | 0 | 100 | 0 | 90 | 0 | 0 | - | 0 | 0 | - | 0 | 0 | 0 | - | 0 | 0 | 0 | 60 |
| 28 | 11.2 | 80 | - | - | - | - | 90 | 98 | 98 | - | - | - | - | 0 | 0 | - | 0 | 0 | - |
| 29 | 11.2 | 40 | - | - | - | - | 70 | 90 | 95 | - | - | - | - | 0 | 0 | - | 0 | 0 | - |
| 30 | 11.2 | 40 | - | - | - | - | 70 | 80 | 60 | - | - | - | - | 0 | 0 | - | 0 | 0 | - |
| 36 | 1.12 | 30 | 100 | 20 | 90 | 100 | 50 | - | 30 | 0 | 0 | 0 | 0 | 0 | - | 0 | 0 | 0 | 80 |

CLAIMS

1. A compound having the formula:

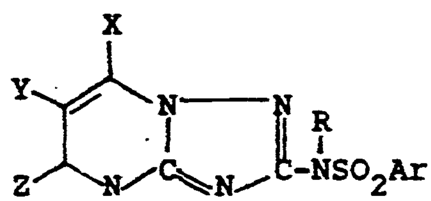

wherein Ar represents a substituted or unsubstituted aromatic or heteroaromatic ring system, R represents hydrogen, alkyl, alkenyl, alkynyl, arylalkyl, substituted arylalkyl, acyl, alkoxycarbonyl, aryloxycarbonyl, dialkylaminocarbonyl, alkylsulfonyl, arylsulfonyl, alkylthiocarbonyl or arylthiocarbonyl and X, Y and Z independently represent hydrogen, alkyl, haloalkyl, hydroxy, alkoxy, haloalkoxy, aryl, substituted aryl, halogen, alkylthio, amino, carboxylic acids and esters or two adjacent substituents are joined together in a cyclic structure, or the pyrimidine ring may be reduced to form a 4,5,6,7-tetrahydro-1,2,4-triazolo[1,5-a]pyrimidine ring.

2. A compound as claimed in Claim 1 wherein Ar is

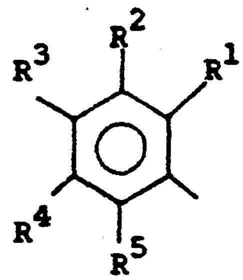

wherein $R^1$ represents halo, $-NO_2$, phenyl, $-CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, -CN, $-COOR^7$, $-CONR^8(R^9)$, $-SO_2NR^8R^9$, $-SO_3R^8$ and $-SO_3CH_2CF_3$; $R^2$ and $R^4$ represent H, halo, $C_1$-$C_4$ alkyl and $-OR^8$; $R^3$ is H; and $R^5$ represents H, $C_1$ to $C_4$ alkyl, halo, $NO_2$, $CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, -CN, $-COOR^7$, $-CONR^8(R^9)$, $-SO_3R^8$, and $-SO_3CH_2CF_3$ wherein $R^6$ represents aryl or $C_1$-$C_4$ alkyl, $R^7$ represents $C_1$-$C_6$ alkyl, alkenyl or alkynyl and $R^8$ and $R^9$ individually represent $C_1$-$C_4$ alkyl; and X, Y and Z represent H, $C_1$-$C_4$ alkyl or $C_1$-$C_4$ alkoxy groups, or X and Y or Y and Z can be joined to form a cyclo alkyl ring or X and Y or Y and Z can be joined to form a ring containing a heteroatom.

3. A compound as claimed in Claim 2 wherein X and Z independently represent hydrogen, $C_1$ to $C_4$ lower alkyl or alkoxy group and Y is hydrogen.

4. A compound as claimed in Claim 2 wherein X and Z are methyl and Y is hydrogen.

5. A compound as claimed in Claim 1 wherein Ar is

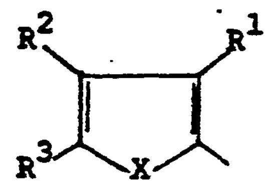

wherein X represents O or S, and $R^1$, $R^2$ and $R^3$ are each independently hydrogen, alkyl or halo.

6. A compound as claimed in Claim 1 wherein Ar is

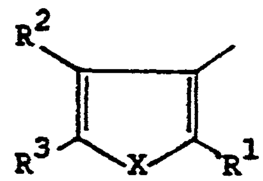

wherein X represents O or S, and $R^1$, $R^2$ and $R^3$ are each independently hydrogen, alkyl, halo or $-COOR^4$ wherein $R^4$ represents $C_1$-$C_6$ alkyl, alkenyl or alkynyl.

7. A herbicidal composition comprising an inert carrier in admixture with a herbicidally effective amount of at least one compound as claimed in any one of Claims 1 to 6.

8. A method of controlling undesired vegetation which comprises the application of a herbicidally effective amount of a compound as claimed in any one of Claims 1 to 6, or of a composition as claimed in Claim 7.

9. A method of preparing a compound as claimed in Claim 1 in which the group R is a hydrogen atom wherein a compound of the formula:

$$ArSO_2Cl$$

wherein Ar represents a substituted or unsubstituted aromatic or heteroaromatic ring system is reacted under basic conditions with a compound of formula:

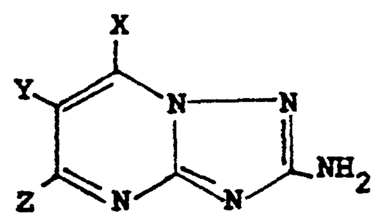

wherein X, Y and Z are each independently hydrogen, alkyl, haloalkyl, alkoxy, haloalkoxy, halogen, or two adjacent substituents are joined together in a cyclic structure.

10. A method as claimed in Claim 9 wherein the reaction is carried out in the presence of pyridine.

11. A method for the preparation of a compound as claimed in Claim 1 in which the group R is as defined in Claim 1 but is other than a hydrogen atom, which comprises reacting a compound of the formula:

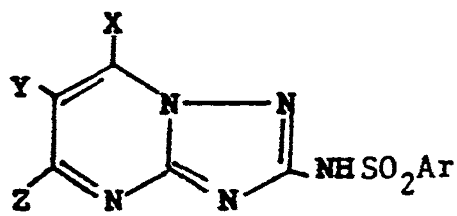

wherein X, Y, Z and Ar are as defined in Claim 1 with an electrophilic reagent of the formula RX, wherein R is as defined above.

12. A method as claimed in Claim 11 wherein the reaction is carried out in the presence of a base and a suitable solvent.

CLAIMS FOR AUSTRIA:

1. A herbicidal composition comprising an herbicidally effective compound of at least one compound having the formula:

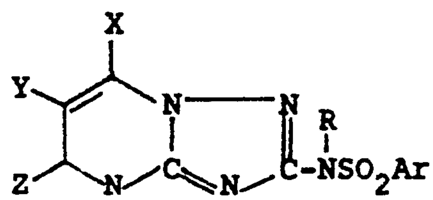

wherein Ar represents a substituted or unsubstituted aromatic or heteroaromatic ring system, R represents hydrogen, alkyl, alkenyl, alkynyl, arylalkyl, substituted arylalkyl, acyl, alkoxycarbonyl, aryloxycarbonyl, dialkylaminocarbonyl, alkylsulfonyl, arylsulfonyl, alkylthiocarbonyl or arylthiocarbonyl and X, Y and Z independently represent hydrogen, alkyl, haloalkyl, hydroxy, alkoxy, haloalkoxy, aryl, substituted aryl, halogen, alkylthio, amino, carboxylic acids and esters or two adjacent substituents are joined together in a cyclic structure, or the pyrimidine ring may be reduced to form a 4,5,6,7-tetrahydro-1,2,4-triazolo[1,5-a]pyrimidine ring; in admixture with an inert carrier.

2. A composition as claimed in Claim 1 wherein Ar is

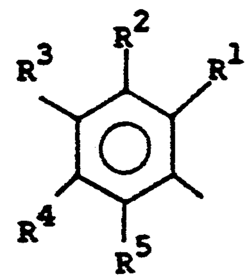

wherein $R^1$ represents halo, $-NO_2$, phenyl, $-CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONR^8(R^9)$, $-SO_2NR^8R^9$, $-SO_3R^8$ and $-SO_3CH_2CF_3$; $R^2$ and $R^4$ represent H, halo, $C_1$-$C_4$ alkyl and $-OR^8$; $R^3$ is H; and $R^5$ represents H, $C_1$ to $C_4$ alkyl, halo, $NO_2$, $CF_3$, $-OCF_3$, $-OCF_2CF_2H$, $-OCF_2CCl_2H$, $-OCH_2CF_3$, $-SCF_3$, $-SCF_2CF_2H$, $-SCF_2CCl_2H$, $-SOCF_3$, $-SOCF_2CF_2H$, $-SOCF_2CCl_2H$, $-SO_2CF_3$, $-SO_2CF_2CF_2H$, $-SO_2CF_2CCl_2H$, $-SR^6$, $-SOR^6$, $-SO_2R^6$, $-CN$, $-COOR^7$, $-CONR^8(R^9)$, $-SO_3R^8$, and $-SO_3CH_2CF_3$ wherein $R^6$ represents aryl or $C_1$-$C_4$ alkyl, $R^7$ represents $C_1$-$C_6$ alkyl, alkenyl or alkynyl and $R^8$ and $R^9$ individually represent $C_1$-$C_4$ alkyl; and X, Y and Z represent H, $C_1$-$C_4$ alkyl or $C_1$-$C_4$ alkoxy groups, or X and Y or Y and Z can be joined to form a cyclo alkyl ring or X and Y or Y and Z can be joined to form a ring containing a heteroatom.

3. A composition as claimed in Claim 2 wherein X and Z independently represent hydrogen, $C_1$ to $C_4$ lower alkyl or alkoxy group and Y is hydrogen.

4. A composition as claimed in Claim 1 wherein X and Z are methyl and Y is hydrogen.

5. A composition as claimed in Claim 1 wherein Ar is

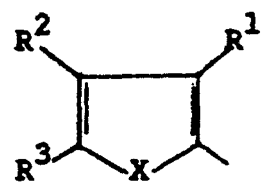

wherein X represents O or S, and $R^1$, $R^2$ and $R^3$ are each independently hydrogen, alkyl or halo.

6. A composition as claimed in Claim 1 wherein Ar is

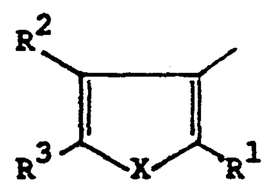

wherein X represents O or S, and $R^1$, $R^2$ and $R^3$ are each independently hydrogen, alkyl, halo or $-COOR^4$ wherein $R^4$ represents $C_1$-$C_6$ alkyl, alkenyl or alkynyl.

7. Method of controlling undesired vegetation which comprises the application of a herbicidally effective amount of a compound as defined in any one of Claims 1 to 6 or of a composition as claimed in any one of Claims 1 to 6.

8. A method of preparing a compound as defined in Claim 1 in which R is other than a hydrogen atom wherein a compound of the formula:

$$ArSO_2Cl$$

wherein Ar represents a substituted or unsubstituted aromatic or heteroaromatic ring system is reacted under basic conditions with a compound of formula:

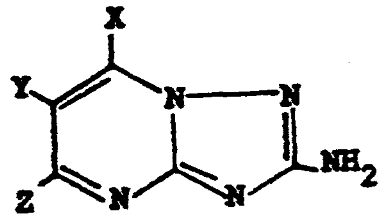

wherein X, Y and Z are each independently hydrogen, alkyl, haloalkyl, alkoxy, haloalkoxy, halogen, or two adjacent substituents are joined together in a cyclic structure.

9. A method as claimed in Claim 8 wherein the reaction is carried out in the presence of pyridine.

10. A method for the preparation of a compound as defined in Claim 1 in which the group R is as defined in Claim 1 but is other than a hydrogen atom, which comprises reacting a compound of the formula:

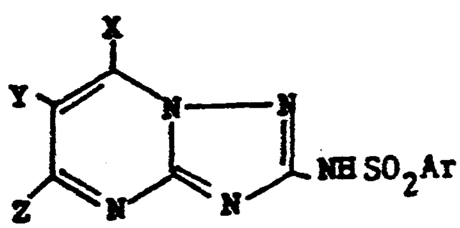

wherein X, Y, Z and Ar are as defined in Claim 1 with an electrophilic reagent of the formula RX, wherein R is as defined above.

11. A method as claimed in Claim 10 wherein the reaction is carried out in the presence of a base and a suitable solvent.